# EUROPÄISCHE PATENTSCHRIFT

(11) **EP 0 819 406 B1**
(45) Veröffentlichungstag und Bekanntmachung des Hinweises auf die Patenterteilung: **27.10.2004**
(21) Anmeldenummer: 97109328.1
(22) Anmeldetag: 09.06.1997
(51) Int. Cl.: A61B 6/03, G01N 23/04, G01N 23/207, G01T 1/29

(54) **Computertomograph**
Computer tomography apparatus
Appareil de tomographie

(30) Priorität: 20.06.1996 DE 19624681
(43) Veröffentlichungstag der Anmeldung: 21.01.1998
(73) Patentinhaber: SIEMENS AKTIENGESELLSCHAFT, 80333 München (DE)
(72) Erfinder: Hupke, Rolf, Dr., 90542 Eckental (DE)

(56) Entgegenhaltungen:
- EP-A- 0 109 205
- EP-A- 0 112 475
- EP-A- 0 365 301
- WO-A-95/20910
- CA-A- 1 245 375
- DE-A- 4 209 376
- US-A- 5 025 463
- US-A- 5 164 973
- US-A- 5 291 402

## Beschreibung

Bei heute in der Medizintechnik verwendeten Computertomographen werden die für ein Schnittbild notwendigen Projektionsdaten aufgenommen, indem ein dünner Fächerstrahl aus dem Strahlenbündel des Röntgenstrahlers ausgeblendet wird und die durch das Objekt transmittierte Strahlung mit einer einzelnen Detektorzeile nachgewiesen wird. Die Länge der einzelnen Detektorelemente in z-Richtung (Richtung der Systemachse) ist dabei so dimensioniert, daß diese die Strahlung für die größte einstellbare Schichtdicke (üblicherweise 10 mm Schicht) aufnehmen können.

Verschiedene Schichtdicken werden durch entsprechende Einstellungen der röhrennahen und einer detektorseitigen Blende erzeugt. Da mit einer derartigen Anordnung nur jeweils Daten für eine Schicht aufgenommen werden können, wird die vom Röntgenstrahler emittierte Röntgenstrahlung nur sehr ineffizient genutzt. Für dreidimensionale Aufnahmetechniken ist das erfaßbare Volumen daher in aller Regel durch die verfügbare Dauerleistung des Röntgenstrahlers beschränkt. Entsprechend lange sind die erforderlichen Aufnahme- bzw. Untersuchungszeiten.

Derartige Beschränkungen des Meßsystems werden weitgehend überwunden, wenn gemäß US-PS 5,291,402 ein Flächendetektor verwendet wird. Ein derartiger Flächendetektor ist ein zweidimensionales Array von Detektorelementen (Mosaik), d. h. er ist von mehreren parallelen Detektorzeilen gebildet, so daß anstelle eines dünnen Fächerstrahles ein auch in z-Richtung ausgedehntes Röntgenstrahlenbündel zur Abbildung verwendet werden kann. Im Gegensatz zu einem konventionellen Einzeilendetektor besteht ein Flächendetektor aus auch in z-Richtung separierten Detektorelementen. Bei einer Drehung des Meßsystems können dann je nach Ausdehnung des Flächendetektors in z-Richtung viele Schichten simultan aufgenommen werden. Benachbarte Zeilen des Flächendetekors erfassen dabei benachbarte Schichten. Die Länge der Detektorelemente in z-Richtung wird daher so gewählt, daß eine Detektorzeile die kleinste, gewünschte Schicht erfaßt.

Aus der EP-A-365 301 ist ein Detektor für einen Computertomographen bekannt, welcher aus mehreren parallelen Detektorzeilen besteht, von denen jede von einer Reihe von Detektorelementen gebildet ist. Der Detektor ist aus einer Reihe von Modulen aufgebaut, von denen jedes mehrere Reihen von Detektorelementen aufweist, die zu unterschiedlichen Detektorzeilen gehören.

Aus der US-A-4 525 628 ist es außerdem bekannt, keramische Szintillatoren als Detektorelemente zu verwenden.

Der Erfindung liegt die Aufgabe zugrunde, einen Computertomographen mit einem Flächendetektor so auszubilden, daß eine einfache Detektorfertigung und eine variable Schichtauswahl ermöglicht werden.

Nach der Erfindung wird diese Aufgabe gelöst durch einen Computertomographen mit einem ein fächerförmiges Röntgenstrahlenbündel aussendenden Röntgenstrahler und einem davon getroffenen Detektor, welcher aus mehreren parallelen Detektorzeilen besteht, von denen jede von einer Reihe von Detektorelementen gebildet ist, wobei das Röntgenstrahlenbündel um eine Systemachse drehbar ist und die bei den verschiedenen Projektionen gebildeten Detektorsignale einem Rechner zugeführt werden, welcher daraus Bilder des untersuchten Objektes berechnet, und wobei der Detektor aus einer Reihe von Modulen aufgebaut ist, von denen jedes Modul von mehreren parallelen Teilmodulen und jedes Teilmodul von einer Reihe von Detektorelementen gebildet ist, wobei die Detektorelemente der Module auf der Basis eines keramischen Szintillators und eines nachgeschalteten Photodetektors aufgebaut sind, und wobei die Szintillatoren aus dem keramischen Basismaterial durch Schlitzen herausgearbeitet sind.

Wesentlich für die Erfindung ist, daß der Detektor aus Modulen, auf der Basis einer Detektorkeramik, aufgebaut ist, die den Erfordernissen entsprechend in ϕ-Richtung, d. h. in Richtung der Drehung der Meßanordnung aus Röntgenstrahler und Detektor strukturiert sind.

Die Erfindung ist nachfolgend anhand der Zeichnung näher erläutert. Es zeigen:
Fig. 1 die wesentlichen Teile eines Computertomographen zur Erläuterung des Erfindungsgedankens und
Fig. 2 bis 6 verschiedene Detektorausbildungen und Detektoreinblendungen zur Erzeugung unterschiedlicher Anzahlen von Schichten und unterschiedlicher Schichtdicken.

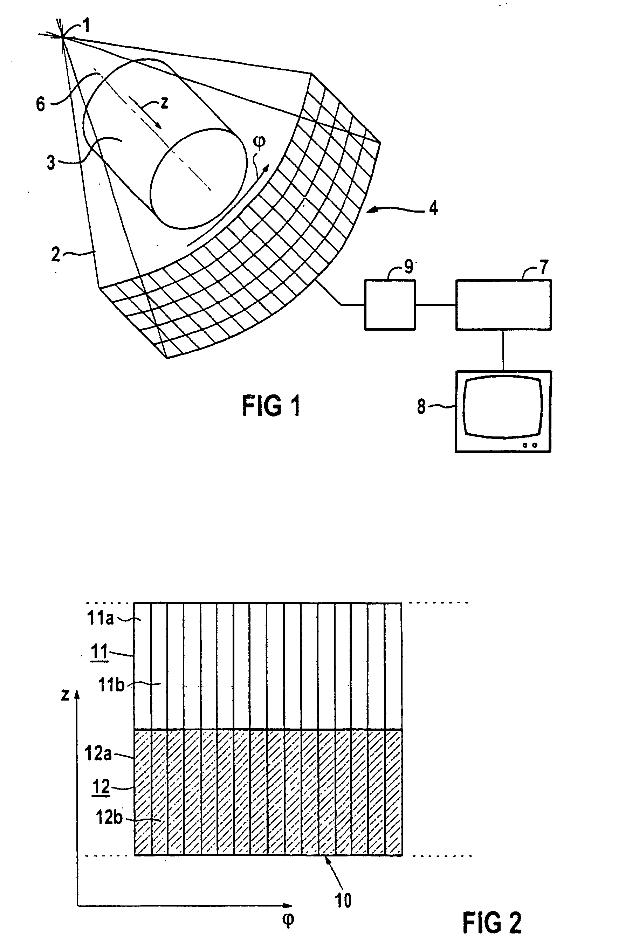

In der Fig. 1 ist der Fokus 1 eines Röntgenstrahlers gezeigt, von dem ein durch eine nicht dargestellte Blende eingeblendetes fächerförmiges Röntgenstrahlenbündel 2 ausgeht, das ein Objekt 3 durchsetzt und auf einem Detektor 4 auftrifft, der aus mehreren parallelen Detektorzeilen besteht, von denen jede von einer Reihe von Detektorelementen gebildet ist. Das Meßsystem 1, 4 ist um eine Systemachse 6 in ϕ-Richtung drehbar, so daß das Objekt 3 unter verschiedenen Projektionen durchstrahlt wird (Axial- und Spiral-Mode). Aus den dabei gebildeten Detektorsignalen berechnet ein Rechner 7 Bilder des Objektes 3, die variabler Schichtdicke entsprechen, welche auf einem Monitor 8 wiedergegeben werden. Die Erfassung der Detektorsignale erfolgt durch einen Multiplexer 9.

Der Detektor 4 ist aus einer Reihe von Modulen aufgebaut, die eine Reihe von keramischen Szintillatoren mit nachgeschalteten Photodioden aufweisen. Die Fig. 2 zeigt eine Ausführung mit einem.Modul 10, das in ϕ-Richtung in sechzehn Elemente und in z-Richtung in zwei Teilmodule 11, 12 geschlitzt ist. Jedes Teilmodul 11, 12 ist demgemäß aus einer Reihe von Detektorelementen 11a, 11b usw. bzw. 12a, 12b usw. aufgeteilt. In ϕ-Richtung wird eine Vielzahl von Modulen entsprechend dem Modul 10 aneinandergereiht. Dadurch ist es möglich, simultan zwei parallele, sich in z-Richtung erstreckende Schichten abzutasten.

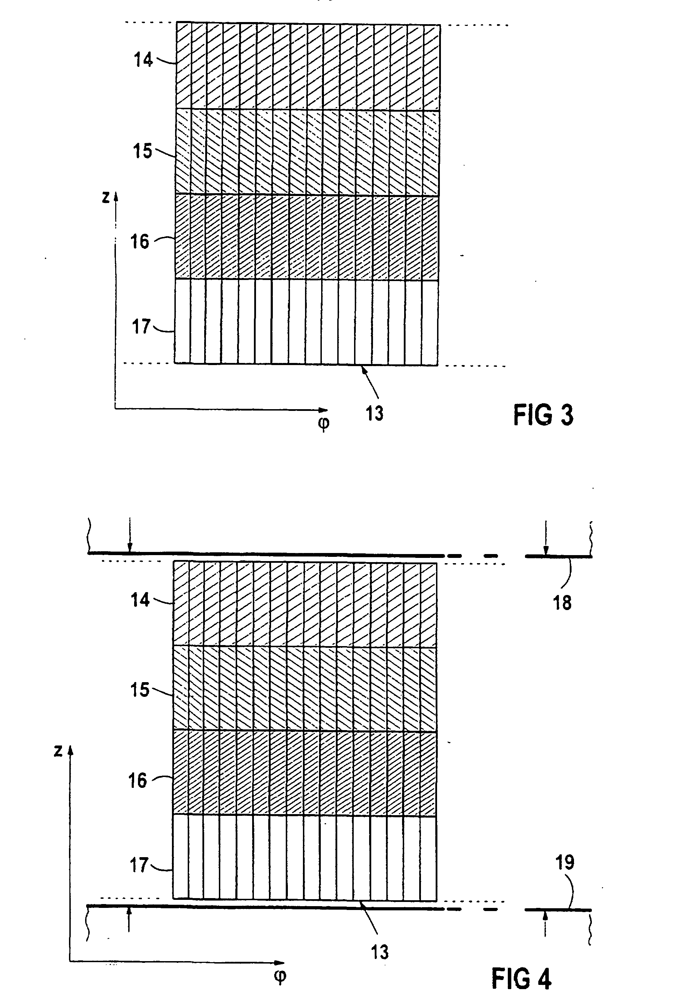

Die Fig. 3 zeigt den Aufbau eines Detektors aus Modulen 13, wobei ein Modul 13 aus vier parallelen Teilmodulen 14 bis 17 aufgebaut ist. Die Teilmodule 14 bis 17 sind in ϕ-Richtung geschlitzt, z. B. ebenfalls in sechzehn Elemente. Dadurch ist es möglich, vier parallele, sich in z-Richtung erstreckende Schichten simultan abzutasten.

Die Fig. 4 zeigt einen Detektoraufbau entsprechend der Fig. 3 mit den zugehörigen, detektornahen Blendenplatten 18, 19. Entsprechend der Stellung der Blendenplatten 18, 19 sind alle vier parallelen Detektorzeilen aus den Teilmodulen 14 bis 17 aktiv und es sind vier Schichten simultan abtastbar. Werden die einander entsprechenden Signale der Detektorelemente der Teilmodule 14 und 15 sowie 16 und 17 zusammengefaßt, so liefern die beiden parallelen Detektorzeilen mit den Teilmodulen 14, 15 und die beiden parallelen Detektorzeilen mit den Teilmodulen 16, 17 jeweils die Signale für eine Schicht, so daß zwei parallele Schichten simultan abtastbar sind.

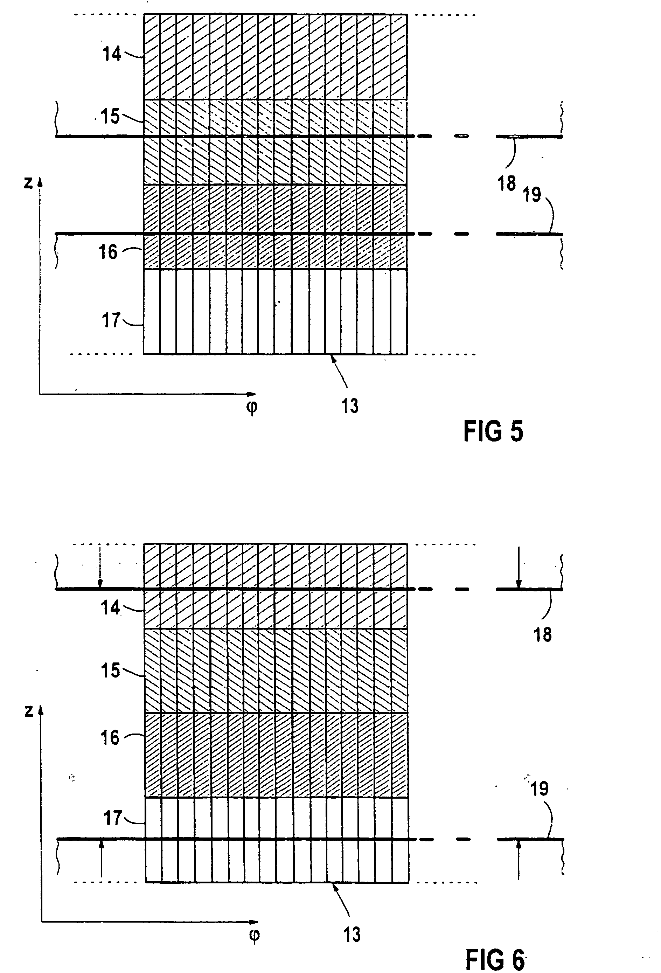

Die Fig. 5 zeigt den Detektor gemäß den Figuren 3 und 4 mit einer Stellung der Blendenplatten 18, 19 in der zwei parallele Schichten abgetastet werden, die durch die Teilmodule 15, 16 definiert sind. Die Teilmodule 14, 17 sind inaktiv. Die Teilmodule 15, 16 sind teilweise durch die Blendenplatten 18, 19 abgedeckt, so daß sich eine geringere Schichtdicke ergibt, als dies durch die Abmessungen der Teilmodule 15, 16 möglich wäre.

Bei der Blendenstellung gemäß Fig. 4 sind beispielsweise vier 5 mm dicke Schichten abtastbar, während bei der Blendenstellung gemäß Fig. 5 zwei 3 mm dicke Schichten abtastbar sind.

Die Fig. 6 zeigt eine Stellung der Blendenplatten 18, 19, in der die Teilmodule 14, 17 teilweise abgedeckt sind. Die Signale einander entsprechender Elemente der Teilmodule 14 und 15 bzw. 16 und 17 werden zusammengefaßt. Dadurch werden zwei parallele Schichten simultan abgetastet, die z. B. jeweils 8 mm dick sind.

Das vorgeschlagene Vier-Zeilen-System erlaubt die Berechnung von vier Bildern pro Umlauf, die entweder der eingestellten Schichtdicke entsprechen oder zu Bildern entsprechend größerer Schichtdicken synthetisiert werden können. Allgemein können N*M mm dicke Schichten simultan abgetastet werden.

Als Detektormaterial kommen alle Röntgenabsorber in Frage, wie z. B. GOS, CdW₀₄, CdTe, GaAs. Aufgrund der einfachen Fertigbarkeit ist GOS besonders gut geeignet.

Der Detektor wird modular gefertigt. Ein Detektor mit den Abmessungen 2*10 mm kann entweder wie in Figur 2 oder aber auch aus demselben Rohling zu einem 4*5 mm-Detektor (Fig. 3) verarbeitet werden. Dies bringt Kostenvorteile, insbesondere bei der benötigten Anzahl von Multiplexern 9 in Fig. 1, wie auch im Fertigungsprozeß. Das vorgeschlagene N-Zeilen-Detektorsystem kann als Grundmodul für aufwendige Meßsysteme mit mehreren Fächerstrahlen dienen.

## Patentansprüche

1. Computertomograph mit einem ein fächerförmiges Röntgenstrahlenbündel (2) aussendenden Röntgenstrahler (1) und einem davon getroffenen Detektor (4), welcher aus mehreren parallelen Detektorzeilen besteht, von denen jede von einer Reihe von Detektorelementen gebildet ist, wobei das Röntgenstrahlenbündel (2) um eine Systemachse (6) drehbar ist und die bei den verschiedenen Projektionen gebildeten Detektorsignale einem Rechner (7) zugeführt werden, welcher daraus Bilder des untersuchten Objektes (3) berechnet, und wobei der Detektor (4) aus einer Reihe von Modulen (10, 13) aufgebaut ist, von denen jedes Modul (10, 13) von mehreren parallelen Teilmodulen (11, 12; 14 bis 17) und jedes Teilmodul (11, 12; 14 bis 17) von einer Reihe von Detektorelementen (11a usw., 12a usw.) gebildet ist, **dadurch gekennzeichnet, dass** die Detektorelemente der Module (10, 13) auf der Basis eines keramischen Szintillators und eines nachgeschalteten Photodetektors aufgebaut sind, und dass die Szintillatoren aus dem keramischen Basismaterial durch Schlitzen herausgearbeitet sind.

2. Computertomograph nach Anspruch 1, bei dem jedes Modul (10) aus zwei parallelen Teilmodulen (11, 12) besteht.

3. Computertomograph nach Anspruch 1, bei dem jedes Modul (13) aus vier parallelen Teilmodulen (14 bis 17) besteht.

4. Computertomograph nach einem der Ansprüche 1 bis 3, bei dem detektornahe Blendenplatten (18, 19) vorgesehen sind, die derart verstellbar sind, daß die Dicke des auf dem Detektor (4) auftreffenden Röntgenstrahlenbündels (2) einstellbar ist.

5. Computertomograph nach Anspruch 4, bei dem die Anzahl der simultan abtastbaren, parallelen Schichten durch Einstellung der Blendenplatten (18, 19) und/oder Zusammenfassung der Ausgangssignale der Detektorelemente wählbar ist.

## Claims

1. A computed tomography apparatus comprising a fan-shaped bundle of X-ray beams (2), an emitting X-ray source (1), and a detector (4) struck by said X-ray beams, constructed from several parallel detector rows, each row being formed from a series of detector elements, wherein the X-ray beams (2) can be rotated about a system axis (6), and the detector signals formed during the various projections are fed into a computer (7), which calculates images of the analysed subject (3), said detector (4) constructed from a series of modules (10,13), of which each module (10, 13) is formed from several parallel sub-modules (11, 12; 14 to 17) and each sub-module (11, 12; 14 to 17) is formed from a series of detector elements (11a, 12a etc.) **characterised in that**, the detector elements of the modules (10, 13) are constructed on the basis of a ceramic scintillator and a subsequently connected photodiode and that said scintillators work their way out of ceramic base materials by means of slits.

2. A computed tomography apparatus according to Claim 1, wherein each of said module (10) consists of two parallel sub-modules (11, 12).

3. A computed tomography apparatus according to Claim 1, wherein said module (13) comprises four parallel sub-modules (14 to 17).

4. A computed tomography apparatus according to one of Claims 1 to 3, wherein diaphragm plates (18, 19) are disposed adjacent to detector, and are thus adjustable, said thickness of the bundle of X-ray beams (2) striking the detector (4) being adjustable.

5. A computed tomography apparatus according to Claim 4, wherein the number of simultaneously scannable parallel slices can be selected by adjusting the diaphragm plates (18. 19) and/or combining the output signal of the detector element.

## Revendications

1. Tomodensitomètre comportant un émetteur (1) de rayons X, émettant un faisceau de rayons X (2) en forme d'éventail, et un détecteur (4) sur lequel tombent les rayons émis par cet émetteur, et qui est constitué de plusieurs lignes de détecteurs parallèles, dont chacune est formée d'une série d'éléments détecteurs, le faisceau de rayons X (2) pouvant tourner autour d'un axe du système (6), et les signaux de détecteurs formés lors des différentes projections étant envoyés à un ordinateur (7), lequel calcule à partir d'eux des images de l'objet examiné (3), le détecteur (4) étant constitué d'une série de modules (10, 13), dont chaque module (10, 13) est formé de plusieurs modules partiels parallèles (11, 12 ; 14 à 17) et chaque module partiel (11, 12 ; 14 à 17) étant formé d'une série d'éléments détecteurs (11a, etc., 12a, etc.), **caractérisé en ce que** les éléments détecteurs des modules (10, 13) sont réalisés sur la base d'un scintillateur céramique et d'un photodétecteur monté en aval, et **en ce que** les scintillateurs sont ménagés par subdivision dans le matériau de base céramique.

2. Tomodensitomètre selon la revendication 1, dans lequel chaque module (10) est constitué de deux modules partiels parallèles (11, 12).

3. Tomodensitomètre selon la revendication 1, dans lequel chaque module (13) est constitué de quatre modules partiels parallèles (14 à 17).

4. Tomodensitomètre selon l'une des revendications 1 à 13, dans lequel sont prévues des plaques formant diaphragme (18, 19), proches du détecteur, qui peuvent être réglées de façon que l'épaisseur du faisceau de rayons X (2) tombant sur le détecteur (4) puisse être ajustée.

5. Tomodensitomètre selon la revendication 4, dans lequel le nombre des couches parallèles pouvant être simultanément balayées peut être choisi par ajustement des plaques formant diaphragme (18, 19) et/ou regroupement des signaux sortants des éléments détecteurs.
